# DEMANDE DE BREVET EUROPEEN

(11) **EP 2 018 892 A1**
(43) Date de publication de la demande: **28.01.2009**
(21) Numéro de dépôt: 08160596.6
(22) Date de dépôt: 17.07.2008
(51) Int. Cl.: A61Q 19/00, A61K 8/99, A61P 17/00, A61K 35/08, A61K 35/74

(54) **Utilisation d'extrait de bactérie cultivée sur eau thermale pour diminuer les poches et/ou les cernes périoculaires**

(30) Priorité: 17.07.2007 FR 0756535
(71) Demandeur: L'Oréal, 75008 Paris (FR)
(72) Inventeur: Breton, Lionel, 78000 Versailles (FR); Gueniche, Audrey, 92500 Rueil Malmaison (FR)
(74) Mandataire: Allab, Myriam

(57) **Abrégé**

La présente invention se rapporte à l'utilisation d'extraits bactériens cultivés sur eau thermale comme agent dans une composition de soin et/ou de maquillage du contour de l'oeil, comme agent destiné à diminuer les poches et/ou les cernes périoculaires.

Préférentiellement, l'invention est un extrait issu de la bactérie *Vitreoscilla filiformis*, en particulier, la souche ATCC 15551, cultivées sur un milieu enrichi en eau de la Roche Posay.

## Description

Le domaine de l'invention est lié au soin et/ou au maquillage du contour des yeux, en particulier au soin et/ou au maquillage des cernes péri-oculaires.

La présente invention se rapporte à l'utilisation d'extraits bactériens cultivés sur eau thermale comme agent dans une composition de soin et/ou de maquillage du contour de l'oeil, comme agent destiné à diminuer les poches et/ou les cernes périoculaires.

Préférentiellement, l'invention est un extrait issu de la bactérie *Vitreoscilla filiformis,* en particulier, la souche ATCC 15551, cultivées sur un milieu enrichi en eau de la Roche Posay.

Le contour de l'oeil, de par sa structure et sa forte innervation, représente un site anatomique particulièrement sensible aux facteurs environnementaux (UV, pollution, tabac...) et physiologiques (fatigue, stress...). Le contour de l'oeil a un rôle esthétique très important puisqu'il est le reflet immédiat de la fatigue, de l'humeur et de l'âge.

Dans l'espèce humaine, les yeux clignent mille fois par jour; l'épiderme qui les entoure est très fin, peu irrigué, tous ces facteurs facilitent l'apparition rapide de rides, ridules, cernes et poches. De plus, afin de permettre une mobilité importante, la peau des paupières est extrêmement fine (0,33 à 0,36 mm, soit 3 à 5 fois moins épaisse que le reste de la peau du visage). Facilement déshydratée et soumise aux agressions extérieures, la peau du contour de l'oeil nécessite des soins particuliers.

Plus ou moins colorés ou marqués, les cernes sont selon le cas l'expression d'une grande fatigue, d'un manque de sommeil, d'une vie stressante, voire d'une maladie. Les cernes ont également une origine vasculaire ou héréditaire.

Les paupières sont le reflet du mode de vie : la chaleur, le stress, le tabac, la chaleur, les UV, les mimiques d'expression entraînent de multiples variations au cours de la journée.

Ces variations concernent la vascularisation, l'hydratation et la turgescence des tissus et expliquent les principales modifications observées : gonflement, cernes, ridules, etc.

La peau du contour de l'oeil est très réactive du fait de sa richesse en cellules inflammatoires (mastocytes) ; ce qui en fait un site sujet à des réactions d'intolérance et d'allergie.

De plus, la peau du contour de l'oeil est particulièrement sensible au rayonnement solaire. L'exposition excessive et sans protection peut entraîner une rougeur, une sensibilité, voire un gonflement par troubles de la microcirculation. A long terme, la peau du contour des yeux subit des phénomènes de photovieillissement.

La vascularisation de surface est peu visible, les capillaires étant de faible débit, mais les vaisseaux des couches sous-cutanées constituent une réserve vasculaire importante pouvant varier au cours de la journée.

Les cernes peuvent être temporaires. Elles correspondent à une congestion vasculaire, transitoire ou permanente qui résulte en une hyperpigmentation de la peau. Elles sont toujours accentuées par le tabac, l'abus d'alcool, le stress et la fatigue.

La formation des cernes est due notamment à un ralentissement de la micro-circulation sanguine, surtout pendant la nuit, conduisant à une accumulation de pigments sanguins dans le tissu conjonctif, surtout visibles au lever le matin.

De plus, le système lymphatique, également ralenti pendant la nuit, entraîne un gonflement des paupières. Ce gonflement, associé au relâchement des tissus liés à l'âge, provoque la formation des poches.

Les cernes et les poches ont toujours été considérées comme inesthétiques et l'on a toujours cherché à les masquer voire à les éliminer.

Il existe notamment des produits de maquillage qui permettent de camoufler ou d'atténuer par effet optique les défauts cutanés tels que les taches, les rides, les ridules. Les compositions de fond de teint par exemple ont généralement pour objet d'unifier le teint ; elles donnent un aspect mat à la peau résultant d'un pouvoir diffusant de la lumière à la surface de la peau. Mais certaines compositions peuvent présenter l'inconvénient de ne pas apporter à la peau un aspect naturel en donnant un aspect poudreux voire plâtreux et d'accentuer les défauts de la peau. Et certaines compositions peuvent être desséchantes à long terme et/ou s'étaler difficilement.

Il subsiste donc un besoin de dissimuler ou d'estomper les défauts du contour des yeux, en particulier les poches et/ou les cernes péri-oculaires, avec des compositions permettant d'obtenir un teint uniforme, homogène, d'aspect naturel, lumineux, vivant, ces compositions présentant par ailleurs un bon confort après application sur la peau.

La Demanderesse a mis en évidence l'effet d'un extrait bactérien cultivé sur une eau thermale sur la régularisation de la vascularisation et, en particulier, un effet avantageux de diminution d'une vasodilatation excessive.

Ainsi, la présente invention se rapporte à l'utilisation d'au moins un extrait de bactérie filamenteuse non photosynthétique et non fructifiante cultivée sur un milieu comprenant au moins une eau minérale et/ou thermale non sulfureuse comme agent destiné à diminuer les poches et/ou les cernes périoculaires.

### Extrait bactérien

L'extrait bactérien utilisé selon l'invention est préparé suivant un procédé comprenant la culture d'au moins une bactérie filamenteuse non photosynthétique et non fructifiante sur un milieu comprenant au moins une eau minérale et/ou thermale non sulfureuse.

Les bactéries utilisées sont des bactéries filamenteuses non photosynthétiques qui comprennent notamment les bactéries appartenant à l'ordre des Beggiatoales, et plus particulièrement les bactéries appartenant aux genres Beggiatoa, Vitreoscilla, Flexithrix ou Leucothrix.

Pour la mise en oeuvre de l'invention, on préfère les bactéries appartenant au genre Vitreoscilla, en particulier pour les bactéries de l'espèce *Vitreoscilla filiformis.*

Ces bactéries dont plusieurs ont déjà été décrites ont généralement un habitat aquatique, et peuvent être trouvées notamment dans des eaux marines ou dans des eaux thermales. Parmi les bactéries utilisables, on peut citer par exemple :
*Vitreoscilla filiformis* (ATCC 15551)
*Vitreoscilla beggiatoïdes* (ATCC 43181)
*Beggiatoa alba* (ATCC 33555)
*Flexithrix dorotheae* (ATCC 23163)
*Leucothrix mucor* (ATCC 25107)
*Sphaerotilus natans* (ATCC 13338)

De préférence, la bactérie est celle correspondant à la souche déposée à l'ATCC sous le n° 15551.

Par eau thermale, on entend une eau chaude ou froide qui est utilisée pour ses vertus thérapeutiques ou pour un usage balnéaire. On peut utiliser une eau thermale ou une eau minérale. En général, une eau minérale est propre à la consommation, ce qui n'est pas toujours le cas d'une eau thermale. Chacune de ces eaux contient, entre autre, des minéraux solubilisés et des oligoéléments. Ces eaux sont connues pour être employées à des fins de traitement spécifique selon les oligo-éléments et les minéraux particuliers qu'elles contiennent.

De préférence, on utilise une eau thermale et/ou minérale qui présente une minéralisation supérieure ou égale à 400 mg/l.

On entend, dans l'invention, par "minéralisation", la somme des concentrations en anions et en cations présents dans l'eau thermale ou minérale. Dans les eaux thermales ou minérales utiles selon l'invention, la minéralisation est généralement comprise entre 400 et 900 mg/l.

L'eau thermale et/ou minérale utilisée selon l'invention peut avoir une minéralisation d'au moins 700 mg/l et, en particulier, une concentration totale en carbonates et en bicarbonates d'au moins 150 mg/l et plus préférentiellement d'au moins 360 mg/l et notamment en carbonate et bicarbonate de sodium supérieure à 2 mg/l. La concentration en oxyde de silicium dans l'eau utilisée dans la composition selon l'invention peut être de préférence d'au moins 6 mg/l et plus préférentiellement d'au moins 9 mg/l.

L'eau thermale ou l'eau minérale utilisée selon l'invention peut être choisie parmi l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizières, l'eau de Neyrac-les-bains, l'eau de Lons le Saunier, les Eaux Bonnes, l'eau de Rochefort, l'eau de Saint Christau, l'eau des Fumades et l'eau de Tercis-les-bains.

Avantageusement, elle est choisie parmi les eaux non sulfureuses telles que l'eau de Vittel, les eaux du bassin de Vichy, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizières, l'eau de Neyrac-les-bains, l'eau de Lons le Saunier, l'eau de Rochefort, l'eau de Saint Christau et l'eau de Tercis-les-bains.

Parmi ces eaux, celles qui présentent une concentration totale en carbonates ou bicarbonates supérieure à 360 mg/l sont l'eau de Vittel, l'eau de la Bourboule, l'eau des Fumades, l'eau d'Enghien-les-bains, l'eau de la Roche-Posay, l'eau du bassin de Vichy, l'eau d'Uriage.

Parmi ces eaux celles qui présentent une concentration en carbonates ou bicarbonates comprise entre 150 mg/l et 360 mg/l sont l'eau de Digne, l'eau de Maizières, l'eau de Rochefort, l'eau de Saint-Gervais-les-bains.

Parmi ces eaux, celles qui contiennent au moins 2 mg/l de carbonate ou bicarbonate de sodium sont l'eau de la Roche Posay, l'eau de Vittel, les eaux du bassin de Vichy, l'eau d'Uriage.

Les eaux contenant au moins 9 mg/l d'oxyde de silicium sont l'eau de la Roche Posay, l'eau de Vittel, les eaux du bassin de Vichy, l'eau d'Uriage.

Les eaux thermales ou minérales convenant particulièrement à la mise en oeuvre de l'invention ont une concentration en ions calcium supérieure ou égale à 100 mg/l, voire à 140 mg/l.

Selon un mode de réalisation avantageux, l'eau thermale ou minérale a une concentration en ions hydrogénocarbonates supérieure ou égale à 300 mg/l. Les hydrogénocarbonates, aussi appelés bicarbonates, sont notamment présents à une concentration supérieure ou égale à 350 mg/l.

Selon un mode de réalisation avantageux, les bactéries sont cultivées sur un milieu comprenant au moins une eau thermale. Celle-ci peut en particulier être choisie parmi l'eau de Vittel, les eaux du bassin de Vichy, l'eau d'Uriage, l'eau de la Roche-Posay, l'eau de la Bourboule, l'eau des Fumades, l'eau d'Enghien-les-bains, les Eaux Bonnes, et notamment parmi l'eau de Vittel, les eaux du bassin de Vichy, l'eau de la Roche-Posay, l'eau de la Bourboule et l'eau d'Enghien-les-bains.

Les eaux permettant d'obtenir un résultat particulièrement avantageux selon l'invention sont notamment choisies parmi l'eau de la Roche Posay et l'eau de Vittel, ou une eau de composition similaire.

L'eau de la Roche Posay est extraite de la source du même nom, il s'agit d'une eau bicarbonatée calcique, silicatée et séléniée. Elle comprend généralement environ 387 mg/l d'ions bicarbonates, environ 140 mg/l d'ions calcium et au moins 4 mg/l de sulfates.

L'eau de Vittel est riche en calcium et sels minéraux (841 mg/l) et contient notamment 202 mg/l de calcium, 402 mg/l de bicarbonates et 336 mg/l de sulfates.

On peut en particulier effectuer une culture dans le milieu suivant :

| **Composition** | **Concentration** |
|---|---|
| Extrait autolytique de levure | 0,5 à 5 g/l |
| Peptone végétale | 0,5 à 5 g/l |
| Glucose anhydre | 0,5 à 7 g/l |
| Micro-éléments de Heller | 0,5 à 5 ml/l |
| CaCl₂, 10 H₂O | 0,010 à 0,200 g/l |

On complète à 1000 ml par de l'eau minérale et/ou thermale éventuellement complétée d'eau distillée ou osmosée.

Parmi les peptones utilisables, on peut citer par exemple la peptone papaïnique de soja.

Ce milieu se distingue des milieux généralement utilisés par l'absence de catalase et de sulfure.

Les micro-éléments de Heller ont été décrits par Heller, Ann Sci. Nat. Biol. Veg. 14 : 1-223 (1953). Il s'agit de mélanges de divers éléments minéraux qui ont été recommandés par Heller, non pas pour la culture des bactéries, mais pour la nutrition des tissus végétaux cultivés *in vitro.*

La culture peut être effectuée à la température appropriée convenant pour l'espèce bactérienne cultivée. Généralement cette température est comprise entre 18 et 40°C suivant les souches. Le pH du milieu de culture est de préférence compris entre 5,5 et 8.

La composition des micro-éléments de Heller, pour 1l d'eau, est la suivante:

| | |
|---|---|
| ZnSO₄,7 H₂O | 1 g |
| MnSO₄,H₂O | 0,076 g |
| CuSO₄,5H₂O | 0,003 g |
| Kl | 0,010 g |
| H₃BO₃ | 1 g |
| AlCl₃,6H₂O | 0,050 g |
| NiCl₂,6H₂O | 0,030 g |

Lesdites eaux thermales ou minérales peuvent représenter tout ou partie de la phase aqueuse du milieu de culture. Elles peuvent ainsi se trouver en mélange en toute proportion avec l'eau, en particulier distillée ou osmosée, présente dans le milieu de culture. Le mélange (i) d'eau thermale et (ii) d'eau osmosée ou distillée pourra être dans un ratio compris entre 0,1% et 100%, notamment de 0,1 à 50, en particulier de 0,1 à 25.

En particulier, un extrait de bactérie filamenteuse convenant à l'utilisation selon l'invention est susceptible d'être obtenu par culture de ladite bactérie sur un milieu de culture qui contient un mélange (i) d'eau osmosée ou distillée et (ii) d'eau thermale, dans un ratio (i)/(ii) compris entre 1 et 100, notamment de 1 à 50, en particulier de 1 à 25.

On utilisera en particulier l'eau thermale de La Roche Posay, telle que définie dans ce qui précède.

Après mélange de tous les éléments du milieu, on procède avantageusement à une stérilisation du milieu de culture contenant l'eau thermale et/ou minérale; cette étape est effectuée par des méthodes connues de l'homme du métier telles que la stérilisation par filtration ou par la chaleur.

Le milieu de culture est ensuite ensemencé par les bactéries.

Les milieux convenant le mieux à la culture des bactéries sont tels que l'eau thermale ou minérale représente de préférence au moins 0,1% de la quantité d'eau introduite pour la préparation du milieu, notamment de 0,1 à 99,9%. De bons résultats sont obtenus avec des concentrations d'eau thermale d'environ 1 ou 2%, notamment d'environ 1,33% par rapport à l'eau osmosée et/ou distillée, par exemple de 0,5 à 20%, voire de 0,5 à 50%, mais ces concentrations peuvent être augmentées sans inconvénient.

De façon connue, le procédé de préparation de l'extrait bactérien comprend au moins une étape dans laquelle on récupère les bactéries à la fin de la culture, en particulier en les séparant du milieu de culture.

Après culture des bactéries, on peut isoler la biomasse par diverses méthodes connues, par exemple par filtration, par coagulation avec un alcool (éthanol, isopropanol, isobutanol), par séchage sur cylindre à précouche (amidon, diatomées...) raclée, ou par lyophilisation. Une concentration préalable, par exemple à 80°C sous pression réduite, améliore cette séparation.

La biomasse peut être utilisée vivante ou bien être traitée par différents procédés. On peut procéder à une opération de rupture des enveloppes, par exemple par l'action des ultrasons. On peut en outre préparer des extraits à l'aide d'un alcool tel que l'éthanol ou le propanol. On peut également préparer des extraits lipopolysaccharidiques selon les méthodes connues, voir par exemple Noris et Ribbons, Methods in Microbiology, Vol. 5B, Academic Press (1971). La méthode généralement utilisée est la méthode bien connue dite de Westphal (ou une méthode apparentée), qui consiste à faire l'extraction avec des mélanges phénol-eau à 65°C. On soumet ensuite l'extrait à une dialyse pour éliminer le phénol.

L'extrait bactérien utile selon l'invention peut encore résulter de la mise en oeuvre du procédé suivant : (i) on cultive au moins une bactérie appartenant à l'ordre des Beggiatoales sur un milieu comprenant un ose comme source principale de carbone et au moins une eau minérale ou thermale, puis (ii) après fermentation, on sépare les bactéries du milieu de culture, pour récupérer ladite masse des bactéries.

Les bactéries récupérées à l'issue de l'étape de fermentation peuvent notamment être soumises à un traitement de stabilisation et/ou d'extraction. C'est l'extrait de bactéries filamenteuses ainsi obtenu qui sera généralement utilisé dans ou pour la préparation de compositions cosmétiques ou dermatologiques. De manière connue en soi, l'extrait pourra ainsi être stérilisé en particulier par filtration ou par autoclavage.

Par extrait de bactéries filamenteuses non photosynthétiques, on entend aussi bien le surnageant de culture desdites bactéries, la biomasse obtenue après culture desdites bactéries ou encore les extraits de la biomasse obtenus par traitement de cette biomasse. Pour préparer l'extrait selon l'invention, on peut cultiver lesdites bactéries selon le procédé selon l'invention, puis les séparer de la biomasse obtenue, par exemple par filtration, centrifugation, coagulation et/ou lyophilisation.

Ainsi, après culture, les bactéries sont concentrées par centrifugation. La biomasse obtenue est autoclavée. Cette biomasse peut être lyophilisée pour constituer ce que l'on appelle l'extrait lyophilisé. Toute méthode de lyophilisation connue de l'homme du métier est utilisable pour préparer cet extrait.

La fraction surnageante de cette biomasse peut également être filtrée dans un récipient stérile pour éliminer les particules en suspension. Cette fraction surnageante peut également être transvasée stérilement dans un récipient stérile. Selon un mode de réalisation particulier de l'invention, la fraction surnageante ainsi obtenue est utilisée à titre d'actif cosmétique ou dermatologique.

L'extrait bactérien utile selon l'invention peut être formulé dans un support approprié à raison d'au moins 20% en poids par rapport au poids total de la composition, en particulier à raison de 0,001 à 20% en poids par rapport au poids total de la composition et plus particulièrement à raison de 0,01 à 10% en poids par rapport au poids total de la composition.

Pour certaines applications ou formulations spécifiques, il peut être avantageux d'utiliser des concentrations pondérales élevées en extrait bactérien, par exemple, comprises entre 15 à 20%.

L'extrait bactérien cultivé sur un milieu enrichi en eau thermale peut encore être utilisé sous forme de fractions de composants cellulaires ou sous la forme de métabolites. Le(s) microorganisme(s), métabolite(s) ou fraction(s) peu(ven)t également être introduit(s) sous la forme d'une poudre lyophilisée, d'un surnageant de culture et/ou le cas échéant sous une forme concentrée.

Pour certaines applications, la biomasse vivante peut être utilisée telle qu'elle par exemple sous forme de masques ou de cataplasme pour produire un effet immédiat.

Au sens de l'invention, le terme "métabolite" désigne toute substance issue du métabolisme des microorganismes considérés selon l'invention et dotée d'une efficacité pour le traitement des cernes.

De manière inattendue, la Demanderesse a constaté que les extraits bactériens cultivés sur eau thermale pouvaient s'avérer efficaces pour réguler les défauts de vascularisation du contour des yeux et ainsi prévenir et/ou réduire les poches et/ou les cernes périoculaires.

En effet, la Demanderesse a pu mettre en évidence que l'extrait de la bactérie *Vitreoscilla filiformis* cultivée sur l'eau thermale de la Roche Posay a une efficacité sur le traitement des désordres vasculaires accrue par rapport à l'extrait de la même bactérie cultivée sur milieu classique, c'est-à-dire sans eau minérale ou thermale.

La différence essentielle entre ces deux extraits réside dans les protocoles de préparation du milieu de culture où il y a substitution, au moins en partie, de l'eau osmosée par l'eau de La Roche Posay. Sans que la Demanderesse ne soit tenue à une quelconque hypothèse, cela conduirait notamment à une modification du métabolisme des bactéries causée par un enrichissement du milieu de culture en éléments minéraux, particulièrement en sélénium, strontium et zinc.

Il est également intéressant de noter que l'introduction de cette biomasse dans un support formulatoire n'entraîne pas de risque de surexposition à ces éléments, Se et Zn sont des éléments essentiels à l'organisme et le Sr est largement répandu dans l'alimentation.

Le tableau ci-dessous fournit les concentrations de ces éléments chimiques dans l'extrait bactérien selon l'invention préparé selon le protocole de l'exemple 1 (extrait lyophilisé).

| | |
|---|---|
| Se (mg/kg) | 6 |
| Sr (mg/kg) | 10 |
| Zn (mg/kg) | 216 |

Ainsi, l'application de cet extrait enrichi entraîne des expositions topiques en sels minéraux par jour de l'ordre de :

| | |
|---|---|
| Se (µg/J) | 0,008 |
| Sr (µg/J) | 0,0032 |
| Zn (µg/J) | 0,094 |

Rappelons ici que l'utilisation des ions pour l'amélioration de l'état cutané est très ancienne. Ainsi, les cures thermales à visée dermatologique sur les bords de la Mer Morte -étendue d'eau la plus saline du monde- remontent à l'Antiquité (Abels DJ et coll, Clinics in Dermatol 14 : 653-658,1996). Ces bains exercent une activité anti-prurigineuse et il n'est pas rare que les personnes traitées éprouvent le sentiment d'avoir une peau plus lisse et souple (Even-Pazz Z, Isr J Med Sci 32 :11-15, 1996). A ce jour, l'intérêt de l'application topique de cations a été autant étudié dans le domaine de la sensibilité que dans celui de la sécheresse cutanée. Parmi les cations divalents, c'est l'effet apaisant du strontium qui a été le plus documenté (Hahn GS, In biochemical modulation of skin reactions. Kydonieus AF, Will JJ (eds), CRC, Boca Raton, Florida, US, 261-272, 2000).

Ainsi, l'invention se rapporte à l'utilisation cosmétique d'au moins un extrait d'une bactérie filamenteuse non photosynthétique et non fructifiante cultivée sur un milieu comprenant au moins une eau minérale et/ou thermale non sulfureuse comme agent destiné à diminuer les poches et/ou les cernes périoculaires.

En outre, l'utilisation selon l'invention permet avantageusement de diminuer l'hyperpigmentation de la zone suborbitale et/ou éclaircir les cernes et d'uniformiser le teint de la zone des cernes avec le reste du visage.

Les extraits bactériens actifs selon l'invention sont capables d'agir sur la composante vasculaire des désordres du contour de l'oeil, en particulier, des cernes et permettre ainsi de réduire le gonflement et/ou normaliser la couleur des cernes.

Par cerne, on entend une zone localisée autour de l'oeil, en particulier en dessous et sur le coté intérieur de l'oeil, présentant un relief qui n'est pas dans la continuité de la peau du visage, c'est-à-dire en creux ou en poche (zone gonflée) et optionnellement avec une couleur et/ou une texture de la peau différente de celle du reste du visage.

Selon une alternative, on peut associer l'extrait bactérien utile selon l'invention avec d'autres agents notamment choisi parmi les rétinoïdes ou les corticostéroïdes, les anti-radicaux libres, les alpha-hydroxy ou alpha-céto acides ou leurs dérivés, ou encore les bloqueurs de canaux ioniques.

Les compositions selon l'invention peuvent, en outre, contenir des additifs inertes ou même pharmacodynamiquement ou cosmétiquement actifs ou des combinaisons des ces additifs et notamment : des agents mouillants, des agents dépigmentant tels que l'hydroquinone, l'acide azelaïque, l'acide caféïque ou l'acide kojique ; des émollients ; des agents hydratants comme le glycérol, le PEG-400, l'urée ; des agents antiséborrhéiques ou antiacnéiques, tels que la S-carboxyméthylcystéine, la S-benzyl-cystéamine, leurs sels et leurs dérivés, ou le peroxyle de benzoyle ; des antibiotiques comme l'érythromycine et ses esters, la néomycine, la clindamycine et ses esters, les tétracyclines ; des agents antifongiques tels que le kétoconazole ou les polyméthylène-4,5 isothiazolinones-3 ; des agents favorisant la repousse des cheveux, comme le Minoxidil (2,4-diamino-6-pipéridino-pyrimidine-3-oxyde) et ses dérivés, le Diazoxide (7-chloro 3-methyl 1,2,4-benzothiadiazine 1,1-dioxyde) et le Phénytoïne (5,4-diphényl-imidazoline 2,4-dione); des agents anti-inflammatoires non stéroïdiens, des carotenoïdes et , notamment le β-carotène ; des agents anti-psoriasiques tels que l'anthraline et ses dérivés et enfin, les eicosa-5,8,11,14-tétraénoïque et eicosa-5,8,11-trynoïque, leurs esters et les amides.

La composition selon l'invention peut également contenir des agents conservateurs, tels que des esters de l'acide parahydroxybenzoïque, les agents stabilisants, des agents régulateur d'humidité, des agents émulsionnants, des filtres UVA et UVB, des anti-oxydants, tels que l'alpha-tocophérol, le butylhydroxyanisole ou le butylhydroxytoluène.

Avantageusement, ledit agent utilisé selon l'invention pourra être incorporé dans un système permettant sa libération au niveau du contour de l'oeil, après application de la composition sur celui-ci.

Ledit extrait bactérien est présent dans la composition en une quantité efficace pour obtenir l'effet recherché, à savoir une diminution des cernes et/ou des poches périoculaires.

Cet effet peut être mesuré par simple observation visuelle ou par analyse comparative d'images.

La composition selon l'invention peut être à usage cosmétique ou dermatologique. Préférentiellement, la composition de l'invention est à usage cosmétique destiné à améliorer l'aspect de la peau, en particulier l'aspect du contour de l'oeil.

Il s'agira en particulier d'une composition de soin et/ou de maquillage des cernes et/ou des poches périoculaires.

Elle peut se présenter sous toutes les formes galéniques normalement utilisées dans les domaines cosmétique et dermatologique, adaptées à la voie orale ou topique, préférentiellement à la voie topique sur la peau.

Sauf indication contraire, dans le cadre de l'invention, par peau, on entend toute surface cutanée du corps incluant la peau et élargie au cuir chevelu et aux muqueuses et semi-muqueuses et par phanères, on entend les cils, poils, cheveux et ongles.

Pour une application topique sur la peau, la composition peut avoir la forme d'une solution aqueuse, hydroalcoolique ou huileuse éventuellement gélifiée, d'émulsion de consistance liquide ou semi-liquide du type lait, obtenue par dispersion d'une phase grasse dans une phase aqueuse (H/E) ou inversement (E/H), d'une émulsion triple (E/H/E ou H/E/H), ou de suspension ou émulsion de consistance molle, semi-solide ou solide de type crème ou gel, d'un produit anhydre liquide, pâteux ou solide ou encore de microémulsions, de microcapsules, de microparticules ou d'une dispersion vésiculaire de type ionique (liposomes ou oléosomes) et/ou non ionique (niosomes) et/ou d'une dispersion de nanosphères.

Ledit agent peut être adsorbé ou incorporé dans des structures particulaires de taille pouvant aller de 1 nm à quelques µm (10 µm), telles que par exemple des microcapsules, microparticules, dispersions vésiculaires de type ionique (liposomes ou oléosomes) et/ou non ionique (niosomes) et/ou dispersions de nanosphères. Ces particules peuvent être avantageusement poreuses et être constituées de silicates ou d'aluminosilicates.

Des exemples de telles formulations sont décrits notamment dans les brevets EP 1 99 636, EP 0 375 520, EP 0 447 318, EP 0 557 489, WO 97/12602, EP 1 151 741 ou US 5,914,126. A titre d'exemple, les microsphères pourront être préparées selon la méthode décrite dans la demande de brevet EP 0 375 520.

Les nanosphères pourront se présenter sous forme de suspension aqueuse et être préparées selon les méthodes décrites dans les demandes de brevet FR0015686 et FR 0101438.

Les oléosomes consistent en une émulsion huile dans eau formée par des globules huileux pourvus d'un enrobage cristal liquide lamellaire dispersé dans une phase aqueuse (voir les demandes de brevet EP 0 641 557 et EP 0 705 593).

L'agent selon l'invention pourra aussi être encapsulé dans des nanocapsules consistant en un enrobage lamellaire obtenu à partir d'un tensio-actif siliconé tel que décrit dans la demande de brevet EP 0 780 115; les nanocapsules pourront également être préparées à base de polyesters sulfonique hydrodispersibles selon par exemple la technique décrite dans la demande de brevet FR 0113337.

On peut également envisager une composition sous forme de mousse ou encore sous forme de spray ou d'aérosol comprenant alors un agent propulseur sous pression.

La composition peut ainsi se présenter sous forme d'une lotion, sérum, lait, crème H/E ou E/H, gel, onguent, pommade, poudre, baume, patch, tampon imbibé, savon, pain, mousse. Une telle composition se présente généralement sous forme fluide ou solide. Par suite une telle composition peut se présenter sous forme fluide, sous forme pâteuse, sous forme semi-solide, et sous forme solide, généralement en stick ou en coupelle, ou avoir été déposée par enduction sur une feuille de papier. Une telle composition peut se présenter sous la forme d'un fond de teint, par exemple sous forme fluide, en pot ou en stick, ou sous forme d'une crème teintée.

En particulier, la composition de soin et/ou de maquillage selon l'invention peut être une crème, un gel, un sérum de soin anti-cernes ou une crème teintée, un fond de teint, de préférence sous une forme solide de type stick comprenant préférentiellement des substances colorantes.

L'invention se rapporte également à une composition comprenant au moins un extrait bactérien utile selon l'invention en association et au moins un autres agents dont l'effet sera de renforcer l'action dudit extrait bactérien par exemple en favorisant la microcirculation cutanée, qui agissent, (i) via une stimulation de la vasodilatation et/ou un effet anti-coagulant et/ou un effet anti-hypertenseur, et/ou (ii) via une stimulation et/ou le maintien de l'angiogénèse, et/ou (iii) via une stimulation de la prolifération des cellules de l'endothélium, et/ou (iv) une stimulation de la migration des cellules de l'endothélium.

En particulier, comme agents qui agissent via (i) une stimulation de la vasodilatation et/ou un effet anticoagulant et/ou un effet anti-hypertenseur, on peut citer :
- des agents anti-hypertenseurs ; en particulier des ouvreurs de canaux potassium ;
- des agents inhibiteurs de phosphodiestérases ;
- des flavonoïdes ou des flavoglycosides ;
- des glucosides ;
- des extraits végétaux aux propriétés vasodilatatrices ;
- des peptides vasodilatateurs non donneurs de NO ;
- d'autres agents vasodilateurs ;
- des agents modulateurs de la température.

### Agents anti-hypertenseurs

On peut citer comme exemples les thiazides ; les inhibiteurs de récepteur de l'angiotensine, comme le losartan ou le candesartan ; les prostaglandines particulièrement de type E et les prostacyclines ; les inhibiteurs ACE, comme la captopril ou le ramipril ; les ouvreurs de canaux potassium, tels que le minoxidil, le cromakalim, le diazoxide, le nicorandil, le pinacidil et dérivés ; les bloqueurs de canaux calcium, tels que la nifedipine, le verapamil, le diltiazem, l'amlodipine ; les bloqueurs de récepteurs adrénergiques, tels que la niacine (acide nicotinique), la prazosine, l'hydralazine; les activateurs des récepteurs de l'acétylcholine muscarique.

Comme agents bloqueurs de canaux calciques, on peut citer par exemple :
- des agents actifs sur la membrane plasmique, complexant le calcium et/ou inhibiteurs de l'entrée du calcium comme les phenylalkylamines comme par exemple le vérapamil, l'anipamil, le gallopamil, le dévapamil, le falipamil, le tiapamil, des dihydropyridines comme par exemple la nifédipine, l'amlodipine, la dazodipine, la félodipine, l'isradipine, lanicardipine, la nimodipine, la nisoldipine, la nitrendipine, la ryosidine, des benzothiazépines comme par exemple le Diltiazem, des diphenylpipérazines comme par exemple la cinnarizine, la flunarizine ; ou
- des agents actifs à l'intérieur de la cellule intervenant sur la libération des réserves intracellulaires du calcium ou alors sur l'inhibition de la formation du complexe calcium/calmoduline. Ce sont par exemple des agents intervenant au niveau du réticulum sarcoplasmique comme par exemple le dantrolène et le TMB-8, des antagonistes de la calmoduline comme par exemple la phénothiazine, la trifluopérazine, la chlorpromazine ou des dérivés du naphtalène ou des anesthésiques locaux comme la dibucaïne ou des antagonistes de la dopamine comme la pimozide, l'halopéridol ou le calmidazolium.

On peut également citer le manganèse et/ou des sels qui bloquent la pénétration du calcium vers le cytoplasme dans de nombreuses cellules.

Comme sels organiques de manganèse, on peut citer le gluconate de manganèse ou le carbonate de manganèse ou l'acétate de manganèse ou le citrate de manganèse ou l'oléate de manganèse ou l'oxalate de manganèse.

Comme sels inorganiques de manganèse on peut citer les sels minéraux comme le chlorure de manganèse ou le borate de manganèse ou le nitrate de manganèse ou le phosphate de manganèse ou le sulfate de manganèse.

De préférence, on utilisera des ouvreurs de canaux potassium, parmi lesquels on peut citer :
- les ouvreurs de canaux potassium ATP-dépendants, tels que le minoxidil, le cromakalim, le diazoxide, le nicorandil, le pinacidil ou cyano-2 (pyridyl-4)-1 (triméthyl-1,2,2 propyl)-3 guanidine de la famille des cyanoguanidines et leurs dérivés ;
- les dérivés de benzopyranne, de benzothiadiazine, d'acide buténoïque, de pyrimidine, ou de pyridine ;
- les composés décrits dans la demande EP 0 886 515 répondant à la formule générale (I) 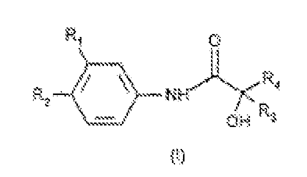 dans laquelle
   R1 est un groupement cyano, un atome d'halogène ou un groupement alkyle, ayant de 1 à 4 atomes de carbone, substitué par au moins un atome d'halogène ;
   R2 est un groupement cyano ou un atome d'halogène ;
   R3 est un groupement alkyle, ayant 1 ou 2 atomes de carbone, éventuellement substitué par au moins un atome d'halogène ;
   R4 est un atome d'hydrogène ou un groupement alkyle, ayant de 1 à 4 atomes de carbone substitué par au moins un atome d'halogène ou un groupement aryle, éventuellement substitué par un ou plusieurs atomes d'halogène, par un ou plusieurs groupements hydroxy, carboxylique, nitro, cyano, alkyle linéaire ou ramifié, ayant 1 à 4 atomes de carbone, alcoxy linéaire ou ramifié, ayant 1 à 4 atomes de carbone, alcanoyle linéaire ou ramifié, ayant 1 à 4 atomes de carbone, perfluoroalkyle ;
   leurs dérivés et/ou leurs sels.

Comme dérivés, on pourra par exemple utiliser les composés décrits dans les demandes EP 0 915 857 et EP 0 916 652.

### Inhibiteurs de phosphodiestérases

On peut citer comme exemples les inhibiteurs de phosphodiestérases de type V tels que la visnadine et l'esculoside, l'icarine et ses dérivés ou des extraits en contenant, tels que décrits dans la demande WO2005/004858.

### Flavonoïdes et flavoglycosides

On peut citer comme exemples les Ginkgo flavoglycosides, l'amentflavone ou des flavones dimériques de Gingko biloba sous forme libre ou complexée à des phospholipides tels que décrits dans la demande WO2005/004858 ; l'hesperidine, l'alpha-G-hespéridine, l'heperidine methyl chalcone, les rutosides (ex : le beta-hydroxyéthyl-rutoside, le trimétrhyl-rutoside).

### Glucosides

On peut citer notamment l'escine, l'escine béta-sitostérol, l'adénosine ou l'ATP (adénosine triphosphate); l'esculoside, l'hesperidine.

### Extraits végétaux

On peut citer par exemple l'extrait d'immortelle de Corse (Helichrysum italicum) tel que décrit notamment dans la demande WO 03/018730 ; les extraits de cassis (Ribes nigrum), de gui, d'épimède (Epimedium grandiflora), de kiwi (Actinidia chinensis L.), de cyprès (Cupressus sempervirens), de mélilot (Melissa officinalis), de petite pervenche (Vinca minora), de Centella asiatica, de Terminalia sericea (séricoside), les extraits de Calendulae, les extraits d'Arnica, les extraits de Ammi visnaga.

### Autres agents vasodilatateurs

On peut citer par exemple l'acide nicotinique (niacine) et ses dérivés, comme les esters d'acide nicotinique, par exemple le xanthinol nicotinate, l'inositol nicotinate ; l'acide salicylique et ses esters ; le dihydro-ergotoxine méthanesulfonate ; le dihydro-ergocornine méthanesulfonate, le dihydro-ergocristine méthanesulfonate, la cinnarizine, la vincamine, la pentoxifylline, le baméthane sulfate, le bencyclane hydrogénofumarate, le béta-pyridylcarbinol.

### Agents modulateurs de la température

Comme autre moyen d'activer la microcirculation cutanée, on peut également moduler la température en utilisant des agents et/ou des formulations à effet thermique (chauffant ou refroidissant).

On connaît par exemple comme composés à effet rafraîchissant, le menthol ou des extraits végétaux et/ou des huiles essentielles de menthe, d'aloe vera ou de ginseng.

Comme exemple de composés à effet réchauffant, on connaît par exemple le camphre, des extraits végétaux ou des huiles essentielles d'eucalyptus ou de gingembre.

Ces composés s'utilisent généralement à des concentrations allant de 0,1% à 10% du poids total de la composition.

### Agents favorisant la stimulation et/ou le maintien de l'angiogénèse

On peut citer notamment :
- les protéases remodelant la matrice extra-cellulaire, qui facilitent la croissance des vaisseaux, comme les collagénases ou les MMPs (matrix metalloproteinases), telles que par exemple MMP-1 ou collagénase interstitielle ; MMP-8 ou collagénase de neutrophile, MMP-13 ou collagénase 3, les gélatinases (ex : MMP-2, MMP-9), les stromélysines (MMP-3) ;
- les facteurs de croissance tels que VEGF (vascular endothelial growth factor); le PDGF (platelet derived growth factor); le b FGF (β fibroblast growth factor); le TGF-β (transforming growth factor TGF-β);
- la leptine et les hormones lipolytiques. Certaines hormones comme l'adénocorticotropine, la mélanocyte-stimulating hormone, l'hormone lutéotropique et le glucagon provoquent une mobilisaton des acides gras libres liée à une vasodilatation. La vasodilatation peut provenir de substances libérées pendant la lipolyse.

### Agents favorisant la stimulation de la prolifération des cellules de l'endothélium

On peut citer notamment :
- les donneurs ou précurseurs de monoxyde d'azote (NO),
- les libérateurs non polymériques de NO;
- les stimulateurs de la synthèse et/ou de l'activité des NO Synthases (NOS).

Des exemples de tels composés sont cités précédemment.

### Agents favorisant la migration des cellules de l'endothélium

On peut citer notamment les peptides dérivés de l'élastine et l'angiotensine II.

Comme actifs cosmétiques adaptés à une utilisation dans les compositions de l'invention, on utilisera notamment des actifs destinés à améliorer l'apparence de la peau, en particulier au niveau du contour de l'oeil et de la zone suborbitale.

Cet actif pourra être notamment choisi parmi un agent dépigmentant, un agent anti-pollution ou anti-radicalaire, un agent tenseur, un agent stimulant la synthèse de macromolécules dermiques ou épidermiques et/ou empêchant leur dégradation, un agent dermorelaxant, et leurs mélanges.

### Agent dépigmentant

Les agents dépigmentants ou anti-pigmentants susceptibles d'être incorporés dans la composition selon la présente invention comprennent par exemple les composés suivants : l'acide kojique ; l'acide ellagique ; l'arbutine et ses dérivés tels que ceux décrits dans les demandes EP 0 895 779 et EP 0 524 109 ; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol ; les dérivés d'iminophénol, en particulier ceux décrits dans la demande WO 99/22707 ; l'acide L-2-oxothiazolidine-4-carboxylique ou procystéine, ainsi que ses sels et esters ; le D-panthétéine sulfonate de calcium, l'acide ascorbique et ses dérivés, notamment le glucoside d'ascorbyle ; et les extraits de plantes, en particulier de réglisse, de mûrier, de scutellaire et de Bacopa monnieri, sans que cette liste soit limitative.

### Agent anti-pollution ou anti-radicalaire

Par l'expression "agent anti-pollution", on entend tout composé capable de piéger l'ozone, les composés aromatiques mono- ou polycycliques tels que le benzopyrène et/ou les métaux lourds tels que le cobalt, le mercure, le cadmium et/ou le nickel. Par "agent anti-radicalaire", on entend tout composé capable de piéger les radicaux libres.

Comme agents piégeurs d'ozone utilisables dans la composition selon l'invention, on peut citer en particulier la vitamine C et ses dérivés dont le glucoside d'ascorbyle ; les phénols et polyphénols, en particulier les tannins, l'acide ellagique et l'acide tannique ; l'épigallocatéchine et les extraits naturels en contenant ; les extraits de feuille d'olivier ; les extraits de thé, en particulier de thé vert ; les anthocyanes ; les extraits de romarin ; les acides phénols, en particulier l'acide chorogénique ; les stilbènes, en particulier le resvératrol ; les dérivés d'acides aminés soufrés, en particulier la S-carboxyméthylcystéine ; l'ergothionéine ; la N-acétylcystéine ; des chélatants comme la N,N'-bis-(3,4,5-triméthoxybenzyl)éthylènediamine ou l'un de ses sels, complexes métalliques ou esters ; des caroténoïdes tels que la crocétine ; et des matières premières diverses comme le mélange d'arginine, ribonucléate d'histidine, mannitol, adénosinetriphosphate, pyridoxine, phénylalanine, tyrosine et ARN hydrolysé commercialisé par les Laboratoires Sérobiologiques sous la dénomination commerciale CPP LS 2633-12F^{®}, la fraction hydrosoluble de maïs commercialisée par la société SOLABIA sous la dénomination commerciale Phytovityl^{®}, le mélange d'extrait de fumeterre et d'extrait de citron commercialisé sous la dénomination Unicotrozon C-49^{®} par la société Induchem, et le mélange d'extraits de ginseng, de pomme, de pêche, de blé et d'orge vendu par la société PROVITAL sous la dénomination commerciale Pronalen Bioprotect^{®}.

Comme agents piégeurs de composés aromatiques mono- ou polycycliques utilisables dans la composition selon l'invention, on peut citer en particulier les tannins tels que l'acide ellagique ; les dérivés indoles, en particulier l'indol-3-carbinol ; les extraits de thé en particulier de thé vert, les extraits de Jacinthe d'eau ou eichornia crassipes ; et la fraction hydrosoluble de maïs commercialisée par la société SOLABIA sous la dénomination commerciale Phytovityl^{®}.

Enfin, comme agents piégeurs de métaux lourds utilisables dans la composition selon l'invention, on peut citer en particulier les agents chélatants tels que l'EDTA, le sel pentasodique d'éthylènediamine tétraméthylène phosphonique, et la N,N'-bis-(3,4,5-triméthoxybenzyl)éthylènediamine ou l'un de ses sels, complexes métalliques ou esters ; l'acide phytique ; les dérivés de chitosan ; les extraits de thé, en particulier de thé vert ; les tannins tels que l'acide ellagique ; les acides aminés soufrés tels que la cystéine ; les extraits de Jacinthe d'eau (Eichornia crassipes) ; et la fraction hydrosoluble de maïs commercialisée par la société SOLABIA sous la dénomination commerciale Phytovityl^{®}.

Les agents anti-radicalaires utilisables dans la composition selon l'invention comprennent, outre certains agents anti-pollution mentionnés précédemment, la vitamine E et ses dérivés tels que l'acétate de tocophéryle ; les bioflavonoïdes ; le co-enzyme Q10 ou ubiquinone ; certaines enzymes comme la catalase, le superoxyde dismutase et les extraits de germes de blé en contenant, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; la guanosine ; les lignanes ; et la mélatonine.

### Agent tenseur

Par "agent tenseur", on entend un composé capable d'exercer une traction sur la peau, qui a pour effet d'estomper temporairement les irrégularités de la surface de la peau, telles que les rides et ridules.

Parmi les agents tenseurs utilisables dans la composition selon la présente invention, on peut citer notamment :
(1) les polymères synthétiques, tels que les latex de polyuréthanne ou les latex acrylique-silicone, en particulier ceux décrits dans la demande de brevet EP-1038519, tels qu'un polydiméthyl siloxane greffé propylthio(polyacrylate de méthyle), propylthio(polyméthacrylate de méthyle) et propylthio(polyacide méthacrylique), ou encore un polydiméthyl siloxane greffé propylthio(polyméthacrylate d'isobutyle) et propylthio(polyacide méthacrylique). De tels polymères siliconés greffés sont notamment vendus par la Société 3M sous les dénominations commerciales VS 80, VS 70 ou LO21,
(2) les polymères d'origine naturelle, notamment (a) les polyholosides, par exemple (i) sous forme d'amidon issu notamment de riz, de maïs, de pomme de terre, de manioc, de pois, de froment, d'avoine, etc... ou (ii) sous forme de carraghénanes, alginates, agars, gellanes, polymères cellulosiques et pectines, avantageusement en dispersion aqueuse de microparticules de gel, et (b) les latex constitués par la résine shellac, la gomme de sandaraque, les dammars, les élémis, les copals, les dérivés cellulosiques, et leurs mélanges,
(3) les protéines et hydrolysats de protéines végétales, en particulier de maïs, de seigle, de froment, de sarrasin, de sésame, d'épautre, de pois, de fève, de lentille, de soja et de lupin, (3) les silicates mixtes, notamment les phyllosilicates et en particulier les Laponites,
(4) les microparticules de cire, choisies par exemple parmi les cires de Carnauba, de Candelila ou d'Alfa,
(5) les particules colloïdales de charge inorganique ayant un diamètre moyen en nombre compris entre 0,1 et 100 nm, de préférence entre 3 et 30 nm, et choisies par exemple parmi : la silice, les composites silice-alumine, l'oxyde de cérium, l'oxyde de zirconium, l'alumine, le carbonate de calcium, le sulfate de baryum, le sulfate de calcium, l'oxyde de zinc et le dioxyde de titane.

### Agent stimulant la synthèse de macromolécules dermiques ou épidermiques et/ou empêchant leur dégradation

Les cellules du derme, en particulier les fibroblastes, produisent des molécules de collagène, d'élastine et de glycoprotéines. Avec l'effet de l'âge ou bien encore sous l'effet des rayons UV, il se produit une diminution notable de ces molécules ainsi qu'une dégradation des fibres de collagène et d'élastine sous l'effet de la collagénase ou de l'élastase.

Parmi les actifs stimulant les macromolécules du derme ou empêchant leur dégradation, on peut citer ceux qui agissent :
- soit sur la synthèse du collagène, tels que les extraits de Centella asiatica ; les asiaticosides et dérivés ; l'acide ascorbique ou vitamine C et ses dérivés, tels que ses sels ou ses esters, en particulier le 5,6-di-O-diméthylsilylascorbate (vendu par la Sté Exsymol sous la référence PRO-AA), le sel de potassuim du dl-alpha-tocopheryl-dl-ascorbyl-phosphate (vendu par la Sté Senju Pharmaceutical sous la référence SEPIVITAL EPC), l'ascorbyl phosphate de magnésium, l'ascorbyl phosphate de sodium (vendu par la Sté Roche sous la référence Stay-C 50) et l'ascorbyl glucoside (vendu par lan société Hayashibara); les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine^{®}; les hormones végétales telles que les auxines et les lignanes ; le palmitoyle de pentapeptide lysine-thréonine-thréonine-lysine-sérine vendu notamment sous la dénomination « MATRIXYL » par la société SEDERMA : le diméthyl amino éthanol ; les extraits de rizhome de Bupleurum Chinensis, tels que ceux vendus sous les dénominations « PLEURIMINCYL », « LIPOCARE » par la société SEDERMA; les hydrolysats de protéine de blé acylés notamment par un groupement palmitoyle, tel que celui vendu sous la dénomination « LIPACID PVB » par la société SEPPIC ; la créatine; le coenzyme Q10 ;
- soit sur la synthèse d'élastine, tels que l'extrait de *Saccharomyces Cerivisiae* commercialisé par la société LSN sous la dénomination commerciale Cytovitin^{®} ; et l'extrait d'algue Macrocystis pyrifera commercialisé par la société SECMA sous la dénomination commerciale Kelpadelie^{®} ; le mélibiose ; les protéines de soja ;
- soit sur la synthèse des glycosaminoglycanes, tels que le produit de fermentation du lait par lactobacillus vulgaris, commercialisé par la société BROOKS sous la dénomination commerciale Biomin yogourth^{®} ; l'extrait d'algue brune Padina pavonica commercialisé par la société ALBAN MÜLLER sous la dénomination commerciale HSP3^{®} ; et l'extrait de Saccharomyces cerevisiae disponible notamment auprès de la société SILAB sous la dénomination commerciale Firmalift^{®} ou auprès de la société LSN sous la dénomination commerciale Cytovitin^{®} ;
- soit sur la synthèse de la fibronectine, tels que l'extrait de zooplancton Salina commercialisé par la société SEPORGA sous la dénomination commerciale GP4G^{®} ; l'extrait de levure disponible notamment auprès de la société ALBAN MÜLLER sous la dénomination commerciale Drieline^{®} ; et le palmitoyl pentapeptide commercialisé par la société SEDERMA sous la dénomination commerciale Matrixil^{®} ;
- soit sur l'inhibition des métalloprotéinases (métalloprotéinases matricielles ou MMP) telles que plus particulièrement les MMP 1, 2, 3, 9. On peut citer : les rétinoïdes et dérivés, les oligopeptides et les lipopeptides, les lipoaminoacides, l'extrait de malt commercialisé par la société COLETICA sous la dénomination commerciale Collalift^{®} ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB^{®}), de trèfle rouge, de lin, de kakkon ou de sauge ;
- soit sur l'inhibition des sérine protéases telles que l'élastase leucocytaire ou la cathepsine G. On peut citer: l'extrait peptidique de graines de légumineuse (Pisum sativum) commercialisé par la société LSN sous la dénomination commerciale Parelastyl^{®} ; les héparinoïdes ; et les pseudodipeptides tels que l'acide {2-[acétyl-(3-trifluorométhyl-phényl)-amino]-3-méthyl-butyrylamino} acétique.

Parmi les actifs stimulant la fillagrine et les kératines, on peut citer notamment l'extrait de lupin commercialisé par la société SILAB sous la dénomination commerciale Structurine^{®} ; l'extrait de bourgeons de hêtre Fagus sylvatica commercialisé par la société GATTEFOSSE sous la dénomination commerciale Gatuline^{®} ; et l'extrait de zooplancton Salina commercialisé par la société SEPORGA sous la dénomination commerciale GP4G^{®}.

De préférence, les agents stimulant la synthèse de macromolécules dermiques ou épidermiques et/ou empêchant leur dégradation sont choisis parmi les extraits de Centella asiatica, l'acide ascorbique et ses dérivés, les peptides extraits de végétaux, tels que l'hydrolysat de soja commercialisé par la société COLETICA sous la dénomination commerciale Phytokine^{®}, l'extrait de Saccharomyces Cerivisiae commercialisé par la société LSN sous la dénomination commerciale Cytovitin^{®} ; l'extrait d'algue brune Padina pavonica commercialisé par la société ALBAN MÜLLER sous la dénomination commerciale HSP3^{®} ; les rétinoïdes et dérivés ; les extraits de romarin ; l'extrait peptidique de graines de légumineuse (Pisum sativum) commercialisé par la société LSN sous la dénomination commerciale Parelastyl^{®} ; l'acide {2-[acétyl-(3-trifluorométhyl-phényl)-amino]-3-méthyl-butyrylamino} acétique ; l'extrait de lupin ; et leurs mélanges.

### Agent myorelaxant ou dermo-décontractant

Les agents myorelaxants ou dermo-décontractants utilisables dans la composition selon l'invention comprennent l'alvérine et ses sels, le gluconate de manganèse, le Diazepam, l'hexapeptide Argireline R commercialisé par la société LIPOTEC, certaines amines secondaires et tertiaires carbonylées, l'adénosine, ainsi que les sapogénines et les extraits naturels, en particulier de Wild Yam, en contenant, ainsi que les extraits de Boswellia serrata.

L'invention se rapporte également à des compositions teintées comprenant l'extrait de bactérie filamenteuse non photosynthétique et non fructifiante cultivée sur un milieu comprenant au moins une eau minérale et/ou thermale non sulfureuse. En particulier, il s'agit d'une composition de maquillage beige pour dissimuler les cernes.

Comme matières colorantes, on peut citer notamment les pigments monochromes, les nacres, les pigments réfléchissants qui émettent une couleur ou n'en émettent pas, les pigments interférentiels, les colorants liposolubles, les colorants hydrosolubles, et leurs mélanges.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D&C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les pigments peuvent avoir subi un traitement de surface.

Selon un mode particulier de l'invention, ledit agent utilisé dans les compositions de l'invention est associé à au moins un autre agent améliorant l'aspect des cernes.

Cet agent est destiné à renforcer l'effet anti-cernes obtenu par le premier agent et/ou conférer à la composition appliquée sur le contour de l'oeil un effet optique anti-cernes et/ou anti-poches, relayé dans le temps par l'effet anti-cernes naturel médié par le premier agent. La présence du premier agent dans la composition permet également de diminuer les concentrations normalement efficaces du second agent pour obtenir l'effet recherché sur les cernes, de sorte à favoriser l'aspect naturel du maquillage.

En particulier, cet agent est choisi parmi :
- un agent agissant sur le système lymphatique ;
- un agent de maquillage destiné à camoufler les cernes autrement nommé agent de camouflage des cernes ;
- et leurs mélanges.

Comme agent agissant sur le système lymphatique, on peut citer notamment des peptides inhibiteurs de l'enzyme ACE (EC3.4.15.1) convertissant l'angiotensine I en angiotensine II, tels que le dipeptide Val-Trp. Ces inhibiteurs de l'enzyme ACE ont pour effet d'augmenter le taux de bradykinine et de favoriser ainsi le drainage lymphatique, tout en diminuant les problèmes de rétention d'eau.

Comme agent de camouflage des cernes, on peut notamment utiliser les charges 'soft focus' ou à effet de flou, les agents fluorescents, les azurants optiques et leurs mélanges.

Par charge « soft-focus », on entend une charge qui en plus donne de la transparence au teint et un effet flou. De préférence, les charges «soft-focus» ont une taille moyenne des particules inférieure ou égale à 15 microns. Ces particules peuvent être de toutes formes et en particulier être sphériques ou non sphériques. De préférence encore, ces charges sont non sphériques.

Les charges « soft-focus » peuvent être choisies parmi les poudres de silice et silicates, notamment d'alumine, les poudres de type polyméthyl méthacrylate (PMMA), le talc, les composites silice/TiO₂ ou silice/oxyde de zinc, les poudres de polyéthylène, les poudres d'amidon, les poudres de polyamides, les poudres de copolymères styrène/acrylique, les élastomères de silicone, et leurs mélanges.

En particulier, on peut citer le talc de taille moyenne en nombre inférieure ou égale à 3 microns, par exemple du talc de taille moyenne en nombre de 1,8 micron et notamment celui vendu sous la dénomination commerciale Talc P3^{®} par la société Nippon Talc, la poudre de Nylon^{®} 12, notamment celle vendue sous le dénomination Orgasol 2002 Extra D Nat Cos^{®} par la société Atochem, les particules de silice traitées en surface par une cire minérale 1 à 2 % (nom INCI: hydrated silica (and) paraffin) telles que celles commercialisées par la société Degussa, les microsphères de silice amorphe, telles que celles vendues sous la dénomination Sunsphère par exemple de référence H-53 par la société Asahi Glass, et les micro-billes de silice telles que celles vendues sous la dénomination SB-700^{®} ou SB-150^{®} par la société Miyoshi, cette liste n'étant pas limitative.

La charge à effet de flou peut être présente dans la composition cosmétique à effet de flou en une teneur allant de 0,1 à 20 % en poids et notamment allant de 1 % à 12 % en poids par rapport au poids total de la composition, notamment entre 5 et 10 %, par exemple de l'ordre de 8 %.

On entend par agent fluorescent une substance qui, sous l'effet de rayons ultraviolet et/ou de la lumière visible, ré-émet dans le visible la portion de lumière qu'elle a absorbé sous la même couleur que celle qu'elle reflète naturellement. La couleur réfléchie naturellement est ainsi renforcée par la couleur ré émise et apparaît extrêmement brillante.

On peut citer par exemple les résines colorées de polyamide et/ou de formaldéhyde/benzoguanamine et/ou de melamine/formaldéhyde/sulfonamide, parmi les cocondensats aminotriazine/formaldéhyde/sulfonamide colorés et/ou parmi les paillettes polyester métallisées et/ou leurs mélanges. Ces pigments fluorescents peuvent aussi se présenter sous la forme de dispersions aqueuses de pigments fluorescents.

On peut encore citer le co-condensat aminotriazine/formaldehyde/sulfonamide fluorescent coloré en rose de taille moyenne des particules 3-4 microns vendu sous la dénomination commerciale « Fiesta Astral Pink FEX-1 » et le co-condensat aminotriazine/formaldehyde/sulfonamide fluorescent coloré en bleu de taille moyenne des particules 3-4,5 microns vendu sous la dénomination commerciale « Fiesta Comet Blue FTX-60 » par la société Swada ou encore la résine benzoguanamine/formaldéhyde recouverte de résine formaldéhyde/urée et colorée en jaune vendue sous la dénomination commerciale « FB-205 Yellow » et la résine benzoguanamine/formaldéhyde recouverte de résine formaldéhyde/urée et colorée en rouge vendue sous la dénomination commerciale « FB-400 Orange Red » par la société UK SEUNG CHEMICAL, la résine polyamide colorée en orange vendue sous la dénomination commerciale « Flare 911 Orange 4 » par la société Sterling Industrial Colors.

Les substances fluorescentes sont de préférence présentes dans la composition, à une teneur allant de 0,1 à 20%, de préférence de 0,1 à 15%, de préférence encore de 0,5 à 3% en poids, par rapport au poids total de la composition.

Lorsque les substances fluorescentes organiques sont blanches, on les appelle également des azurants optiques.

L'azurant optique a pour effet d'intensifier l'éclat et aviver les teintes des compositions cosmétiques les comprenant à l'application sur la peau.

Parmi les azurants optiques, on peut plus particulièrement citer les dérivés du stilbène, en particulier les polystyrylstilbènes et les triazinstilbènes, les dérivés coumariniques, en particulier les hydroxycoumarines et les aminocoumarines, les dérivés oxazole, benzoxazole, imidazole, triazole, pyrazoline, les dérivés du pyrène et les dérivés de porphyrine et/ou leurs mélanges.

De tels composés sont par exemple disponibles sous les dénominations commerciales Tinopal SOP^{®} et Uvitex OB^{®} auprès de la société CIBA GEIGY.

Les azurants optiques préférentiellement utilisés sont le 4,4'-bis[(4,6-dianilino-1,3,5-triazin-2-yl)amino]stilbène-2,2'-disulfonate de sodium, le 2,5 thiophène di-yl bis(5 ter-butyl-1,3 benzoxazole), le di-styryl-4,4' biphényle sulfonate di-sodique et/ou leurs mélanges.

Des exemples de tels composés sont décrits précédemment dans la description.

L'invention concerne également un procédé cosmétique visant à améliorer l'aspect du contour de l'oeil, caractérisé en ce que l'on applique sur le contour de l'oeil et en particulier la zone suborbitale une composition telle que définie précédemment.

Ce procédé est notamment destiné à diminuer les poches et/ou les cernes péri-oculaires, en particulier les cernes.

La composition de l'invention sera appliquée de préférence sur les cernes ou sur les personnes présentant des cernes.

La composition selon l'invention peut être appliquée quotidiennement comme soin / maquillage de jour ou comme soin de nuit.

### Exemple 1 - Préparation d'un extrait bactérien selon l'invention : biomasse de Vitreoscilla filiformis cultivée sur un milieu enrichi en eau thermale de La Roche Posay

Préparation du milieu de culture :

| Composition : | |
|---|---|
| * Extrait de levure | 2 à 3 g |
| * Peptone Papaïnique de soja | 2 à 3 g |
| * Glucose | 2 à 3 g |
| * Micro élément de Heller | 2 ml |
| * CaCl₂, 2H2O | 66.21 mg |
| * Eau thermale La Roche Posay | 13 -14 ml. |

Cette solution mère sera diluée par de l'eau osmosée dans un rapport de 1/75 avant stérilisation.

Le pH du milieu est ajusté à 5,00 par d'une solution molaire H₃PO₄. Le milieu est stérilisé par autoclavage à 121°C pendant 30 minutes. Après refroidissement à température ambiante, le pH est réajusté à 7,20 par rajout d'une solution molaire de KOH.

### Culture :

Après ensemencement à 1% du milieu avec la souche *Vitreoscilla filiformis* déposée à l'ATCC sous le n°15551, la culture est mise en agitation orbitale à 100 tours / min et à 26°C. Après 48 heures de croissance, la culture est centrifugée à 8000 g pendant 15 minutes. Les culots sont récupérés puis autoclavés à 121°C pendant 30 minutes. Cette biomasse peut être utilisée pour des tests d'évaluation.

### Exemple 2 - étude clinique

### Données cliniques :

Une première étude clinique (étude 1), en double aveugle, a évalué en intra-individuel l'effet comparatif d'une crème contenant 5% d'extrait de *Vitreoscilla filiformis* (V. f.) cultivée de façon classique (ci-après dénommé « extrait classique » sur les rougeurs survenant chez des sujets atteints de dermatites atopiques légères à modérées (lésions symétriques contre placebo).

L'extrait dit classique de *Vitreoscilla filiformis* est préparé selon les modalités suivantes :
- *Préparation du milieu de culture :*

| Composition : | |
|---|---|
| * Extrait de levure | 2 g |
| * Peptone Papaïnique de soja | 2 g |
| * Glucose | 2 g |
| * Micro éléments de Heller | 2 ml |
| * CaCl₂, 2H2O | 66.21 mg |
| * Eau | qsp 1 I |

Le pH du milieu est alors ajusté à 5,00 par d'une solution molaire H₃PO₄. Le milieu est stérilisé par autoclavage à 121°C pendant 30 minutes. Après refroidissement à température ambiante, le pH est réajusté à 7,20 par rajout d'une solution molaire de KOH.
- *Culture :*
Au laboratoire : Après ensemencement à 1% du milieu avec la souche *Vitreoscilla filiformis,* la culture est mise en agitation orbitale à 100 tours / min et à 26°C. Après 48 heures de croissance, la culture est centrifugée à 8000 g pendant 15 minutes. Les culots sont récupérés puis autoclavés à 121°C pendant 30 minutes. Cette biomasse peut être utilisée pour des tests d'évaluation.

En fermenteur : dans un fermenteur équipé préférentiellement d'un draft tube pour limiter le cisaillement, on ensemence la souche de *Vitreoscilla filiformis* à 1% volume minimum. Le pH est maintenu stable à 7 UpH durant toute la culture, la T° est régulée entre 26 et 28°C et l'oxygénation maintenue à 10% pO2 durant toute la culture par action soit sur la vitesse d'agitation soit par régulation du débit d'air. Ce type de culture peut être menée en batch, fed-batch ou en continu. Nous préférerons cette dernière technique qui garantit une biomasse reproductible grâce au contrôle du taux de croissance (µ). La biomasse récoltée en continu par centrifugation à 10 000 g est congelée à -20°C. Lorsque la cuve de congélation est pleine, elle est décongelée à 4°C puis conditionnée dans des emballages manipulables par un opérateur. Ces emballages contenant la biomasse sont alors stérilisés pour être stabilisés. Chaque opération de stérilisation représente alors un lot de fabrication.

Dans le cadre de cette étude, la composition comprenant l'extrait bactérien dit « extrait classique » est appliqué 2 fois par jour ont été très bien tolérés.

L'extrait est formulé dans la composition 1A qui est une formule contenant 5% de l'extrait classique dans une émulsion huile dans eau/Arlacel/Myrj contenant 5% Parleam et 15% de silicone volatile. L'effet de cette composition 1A est comparé à celui d'un placébo : la composition 2A qui correspond à l'excipient : émulsion huile dans eau/Arlacel/Myrj contenant 5% Parleam et 15% de silicone volatile.

Cette composition 1A contenant l'extrait « classique » de *Vitreoscilla filiformis* à 5% n'a pas d'effet significatif sur la composante vasculaire des désordres de la peau des sujets testés. A contrario, dans une seconde étude (étude 2 réalisée dans les mêmes conditions que la précédente par la même équipe d'expérimentateurs), la composition contenant 5% d'extrait de *Vitreoscilla filiformis* cultivé sur un milieu enrichi en eau thermale de La Roche Posay (préparé selon l'exemple 1) à démontré également une efficacité spécifique sur la composante vasculaire de l'affection.

L'extrait est formulé dans la composition 1 B qui est une formule contenant 5% d'extrait bactérien selon l'invention (obtenu selon l'exemple 1) dans une émulsion huile dans eau déminéralisée Arlacel/Myrj contenant 5% Parleam, 15% de cyclopentasiloxane, 3% glycérine et 2% vaseline. L'effet de cette composition 1B est comparé à celui d'un placébo : la composition 2B qui correspond à une émulsion huile dans eau de La Roche Posay Arlacel/Myrj contenant 5% Parleam, 15% de cyclopentasiloxane, 3% glycérine et 2% vaseline.

Dans cette étude, les compositions sont appliquées 2 fois par jour, l'efficacité a été observée dans les 15 jours qui ont suivi l'application.

Ainsi, les extraits bactériens cultivés sur l'eau de la Roche Posay de la composition 1 B, contrairement aux extraits bactériens connus, **ont significativement diminué les manifestations cutanées associées à un désordre vasculaire** (oedème) des sujets sur les zones où ils ont été traités en comparaison à l'effet du placebo en controlatéral (p=0, 02, Test de Fisher).

Cette supériorité d'efficacité entre l'étude 1 et l'étude 2 est liée à une spécificité à agir sur la composante vasculaire des manifestations cutanées chez les individus testés.

En effet les extraits bactériens cultivés sur l'eau de la Roche Posay (extrait de l'exemple 1) ont significativement diminué les signes et les symptômes du à une vasodilatation des patients en comparaison à l'effet du placebo en controlatéral (p=0, 02, Test de Fisher).

### Exemple 3 - formulations

| Emulsion contour des yeux anti-cernes | |
|---|---|
| Extrait bactérien de l'exemple 1 | 1% |
| Charge soft focus | 20% |
| Vaseline | 4,00 % |
| Tristéarate de sorbitan | 0,90 % |
| Myristate de Myristyl | 2,00 % |
| Méthylparaben | 0,25 % |
| Eau | qsp 100% |

La charge soft focus contient 3% de kaolin (kaolin colloïdal USPBC de WCD linternational) ; 8% de microsphères de silice (Silica Beads SB700 par la Société MYOSHI) et 10% de séricite (BC281 par WHITTAKER).

| Stick de fond de teint anti-cernes | |
|---|---|
| - Extrait bactérien de l'exemple 1 | 3,00 |
| - dipeptide Val-Trp (agit sur système lymphatique) | 0,5 |
| - Huile de jojoba hydrogénée (Desert Whale) | 2,0 |
| - Résine triméthylsiloxysilicate (KF-73125 de Shin Etsu) | 10,0 |
| - Cire de polyéthylène (Polywax 500) | 15,0 |
| - Oxyde de fer | 3,1 |
| - Oxyde de titane | 10,9 |
| - Stéaryldiméthicone | 7,0 |
| - Séricite (S152 de chez Myoshi) | 6,0 |
| - Cyclopenta(diméthylsiloxane) (viscosité 4 cst) | qsp 100 |

## Revendications

1. Utilisation cosmétique d'au moins un extrait d'une bactérie filamenteuse non photosynthétique et non fructifiante sur un milieu comprenant au moins une eau minérale et/ou thermale non sulfureuse comme agent destiné à diminuer les poches et/ou les cernes périoculaires.

2. Utilisation selon la revendication 1, **caractérisée en ce que** ladite bactérie est *Vitreoscilla filiformis.*

3. Utilisation selon la revendication 1 ou 2, **caractérisée en ce que** ladite eau est l'eau de La Roche Posay.

4. Utilisation selon l'une quelconque des revendications précédentes, **caractérisée en ce que** l'extrait est présent à raison de 0,001 à 20% en poids par rapport au poids total de la composition.

5. Utilisation selon l'une quelconque des revendications 1 à 4, **caractérisée en ce que** ledit agent est destiné à diminuer l'hyperpigmentation de la zone suborbitale et/ou éclaircir les cernes.

6. Utilisation selon l'une quelconque des revendications 1 à 4, **caractérisée en ce que** ledit agent est destiné à uniformiser le teint de la zone des cernes avec le reste du visage.

7. Utilisation selon l'une quelconque des revendications 1 à 6, **caractérisée en ce que** la composition est sous la forme d'une crème contour des yeux anti-cernes, d'un gel contour des yeux anti-cernes, d'un stick anti-cernes.

8. Compositions comprenant d'au moins un extrait de bactérie filamenteuse non photosynthétique et non fructifiante cultivée sur un milieu comprenant au moins une eau minérale et/ou thermale avec un agent actif choisi parmi :
- les agents anti-hypertenseurs
- les agents bloqueurs de canaux calciques ;
- les ouvreurs de canaux potassium ;
- les inhibiteurs de phosphodiestérases ;
- les flavonoïdes et flavoglycosides ;
- les glucosides ;
- les extraits végétaux choisis parmi l'extrait d'immortelle de Corse (Helichrysum italicum) ; les extraits de cassis (Ribes nigrum), de gui, d'épimède (Epimedium grandiflora), de kiwi (Actinidia chinensis L.), de cyprès (Cupressus sempervirens), de mélilot (Melissa officinalis), de petite pervenche (Vinca minora), de Centella asiatica, de Terminalia sericea (séricoside), les extraits de Calendulae, les extraits d'Arnica, les extraits de Ammi visnaga ;
- les agents modulateurs de la température ;
- les agents favorisant la stimulation et/ou le maintien de l'angiogénèse ;
- les agents favorisant la stimulation de la prolifération des cellules de l'endothélium ;
- les agents favorisant la migration des cellules de l'endothélium ;
- les agents dépigmentant ou anti-pigmentants ;
- les agents anti-pollution choisis parmi tout composé capable de piéger l'ozone, les composés aromatiques mono- ou polycycliques tels que le benzopyrène et/ou les métaux lourds ;
- les agents anti-radicalaire ;
- les agents tenseurs ;
- les agents stimulant la synthèse de macromolécules dermiques ou épidermiques et/ou empêchant leur dégradation choisi parmi ceux agissant sur la synthèse du collagène et/ou sur la synthèse d'élastine et/ ou sur la synthèse des glycosaminoglycanes et/ou sur la synthèse de la fibronectine, tels que l'extrait de zooplancton Salina commercialisé par la société SEPORGA sous la dénomination commerciale GP4G^{®} ; et/ou sur l'inhibition des métalloprotéinases (métalloprotéinases matricielles ou MMP) et/ou sur l'inhibition des sérine protéases ou ceux stimulant la fillagrine et les kératines ;
- les agents myorelaxants ou dermo-décontractant.

9. Compositions de maquillage comprenant d'au moins un extrait de bactérie filamenteuse non photosynthétique et non fructifiante cultivée sur un milieu comprenant au moins une eau minérale et/ou thermale avec un agent actif choisi parmi :
- un agent agissant sur le système lymphatique ;
- un agent de maquillage destiné à camoufler les cernes autrement nommé agent de camouflage des cernes ;
- et leurs mélanges.

10. Composition selon la revendication 8 ou 9 **caractérisée en ce qu'**elle se présente sous la forme d'une crème contour des yeux anti-cernes, d'un gel contour des yeux anti-cernes, ou d'un stick anti-cernes.

11. Composition selon l'une quelconque des revendications 8 à 10, **caractérisée en ce que** ladite bactérie est *Vitreoscilla filiformis* (souche ATCC 15551).

12. Composition selon l'une quelconque des revendications 8 à 11, **caractérisée en ce que** ladite eau est l'eau de La Roche Posay.

13. Composition selon l'une quelconque des revendications 8 à 12, **caractérisée en ce que** l'extrait est présent à raison de 0,001 à 20% en poids par rapport au poids total de la composition.

14. Procédé cosmétique visant à améliorer l'aspect du contour de l'oeil, **caractérisé en ce que** l'on applique sur le contour des yeux une composition telle que définie dans l'une des revendications 8 à 13.

15. Procédé selon la revendication 14, **caractérisé en ce que** la composition est appliquée sur les cernes et/ou sur les personnes présentant des cernes.

16. Procédé selon la revendication 14 ou 15, **caractérisé en ce que** ladite bactérie est *Vitreoscilla filiformis* (souche ATCC 15551).

17. Procédé selon l'une quelconque des revendications 14 à 16, **caractérisé en ce que** ladite eau est l'eau de La Roche Posay.

18. Procédé selon l'une quelconque des revendications 14 à 17, **caractérisé en ce que** l'extrait est présent à raison de 0,001 à 20% en poids par rapport au poids total de la composition.
